# EUROPEAN PATENT SPECIFICATION

(11) **EP 0 589 506 B1**
(45) Date of publication and mention of the grant of the patent: **04.12.1996**
(21) Application number: 93202583.6
(22) Date of filing: 03.09.1993
(51) Int. Cl.: A61M 25/06

(54) **Needle holding device for penetration of a needle into the body, such as a needle for intravenous infusion, dialysis and similar uses**
Nadelhalterungsvorrichtung zum Einstechen einer Nadel in den Körper, insbesondere für intravenöse Infusionen, Dialyse und ähnliche Anwendungen
Dispositif de maintien d'aiguille pour la pénétration de cette aiguille dans le corps, en particulier pour perfusions intraveineuses, dialyse et applications similaires

(30) Priority: 24.09.1992 IT RE920075
(43) Date of publication of application: 30.03.1994
(73) Proprietor: C.G.M. S.P.A., I-42015 Corregio (Reggio Emilia) (IT)
(72) Inventor: Parmigiani, Corrado, I-42015 Correggio (Reggio Emilia) (IT)
(74) Representative: Corradini, Corrado

(56) References cited:
- EP-A- 0 023 580
- EP-A- 0 197 180
- EP-A- 0 353 916
- WO-A-92/14502
- US-A- 4 888 001

## Description

This invention relates to a needle holding device for needles which penetrate into the body, in particular for needles for intravenous infusion, dialysis and similar uses.

Needles for intravenous infusion, dialysis etc. are known comprising a pointed front portion which projects from a member into which the rear portion of the needle is inserted. Said member is provided with two fins by which the needle can be held by the fingers and manipulated, particularly for its introduction into the patient's body in a direction tangential to the body surface. At its rear end the needle is connected to a tube feeding the substance to be introduced into the patient.

The present invention relates to the problem of protection against the possibility of being pricked by such needles, particularly after they have already been inserted into a patient's body. In this respect, if such needles prick or scratch a second person after having been used on a first person, they can become a vehicle for the transmission of disease.

There is therefore a considerable danger connected with the manipulation of these needles particularly by those persons who usually work with them, and in particular hospital workers. The disposal of such used needles as refuse also implies danger for those persons handling the refuse containers and for those who come into close proximity with such containers.

For these reasons it is usual to cover the point of such needles after use by mounting on them a thin cap, such as that which was used to protect the needle prior to its use; however, mounting said cap on the used needle is an operation which requires a certain accuracy, and has itself been the cause of pricking. Another drawback is that after its removal from the needle, the cap can easily be mislaid as it is of relatively small dimensions.

Attempts to obviate the said problems have been made by means of devices described in US-A-4888001 and EP-A-0353916.

The object of the present invention is to obviate said problems within the framework of a constructionally economical and effective solution.

Said object is attained by the needle holding device of the invention as defined in the claims.

The invention is described in detail hereinafter with reference to the accompanying figures, which illustrate two different embodiments thereof.
Figure 1 is an enlarged plan view of a first embodiment of the invention in its extended configuration.
Figure 2 is a side view of Figure 1.
Figure 3 is a section on the plane III-III of Figure 1.
Figure 4 is a section on the plane IV-IV of Figure 1.
Figure 5 is a side view, as in Figure 2, showing the device in its first operating configuration.
Figure 6 is a section on the plane VI-VI of Figure 5.
Figure 7 is a front view, as in Figure 1, showing the device in its second configuration.
Figure 8 is a side view, as in Figures 2 and 5, showing the device further folded compared with Figure 7.
Figure 9 is a section on the plane IX-IX of Figure 8.
Figure 10 is a plan view of a second embodiment of the invention in its open configuration.
Figure 11 is a section on the plane XI-XI of Figure 10.
Figure 12 shows the same section as Figure 11, but in its first configuration.
Figure 13 is a side view of Figure 10 in its second configuration.
Figure 14 is a section on the plane XIV-XIV of Figure 13 in which the element 10 is further folded.

In the figures, the needle holding device of the invention is indicated overall by 1, while 3 indicates the needle for penetration into the body and comprises a front portion 31 provided with a point and a rear portion 32 which is connected to a tube 4 feeding the needle 3.

The device 1 comprises a front element 10 positioned adjacent to the front portion 31 but not constrained thereto; the element 10 can be folded on itself substantially about a first folding axis A parallel and adjacent to the portion 31. Means (described in detail hereinafter) are also provided for constraining the rear portion 32 to the front element 10 so that the needle 3 can rotate with respect to the element 10 substantially about a second axis B substantially perpendicular to the needle 3.

The term "folding axis" means either a true axis of rotation about which two rigid bodies rotate (in the case of two rigid bodies articulatedly connected together) or a more or less wide folding region extending axially along said axis, along which a body deforms by flexure (in the case of a single flexible body).

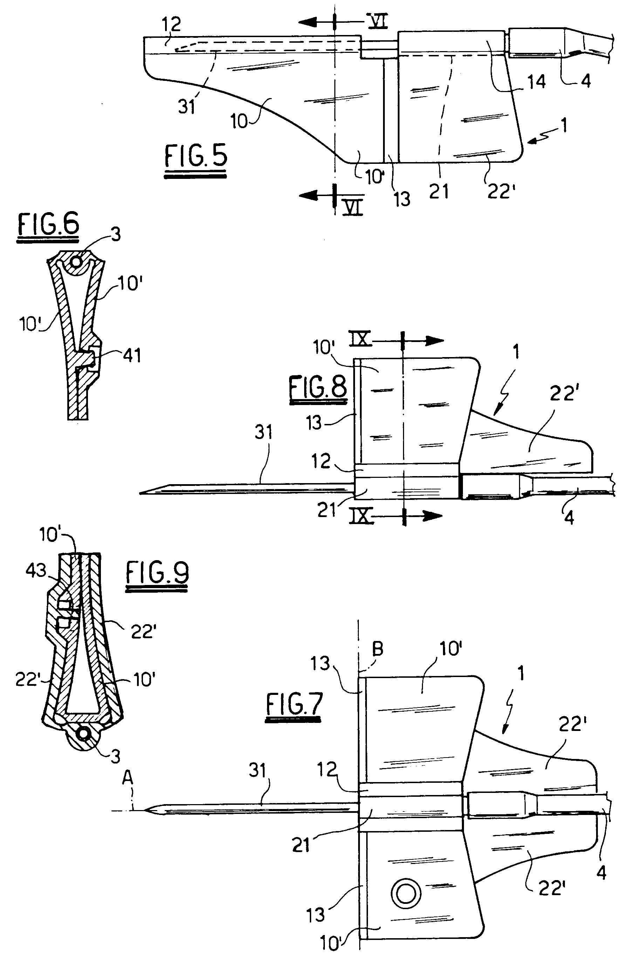

The device 1 can assume a first operating configuration (Figures 5, 6, 12) in which the element 10 is folded on itself by being folded about the first axis A, the front portion 31 being enclosed between the two lateral fins 10' of the element 10 (ie the two portions of the element 10 separated by the first axis A), which face each other in mutual contact; the form and dimensions of the element 10 with reference to the needle 3 are such that when in this configuration the point of the needle 3 does not project beyond the element 10.

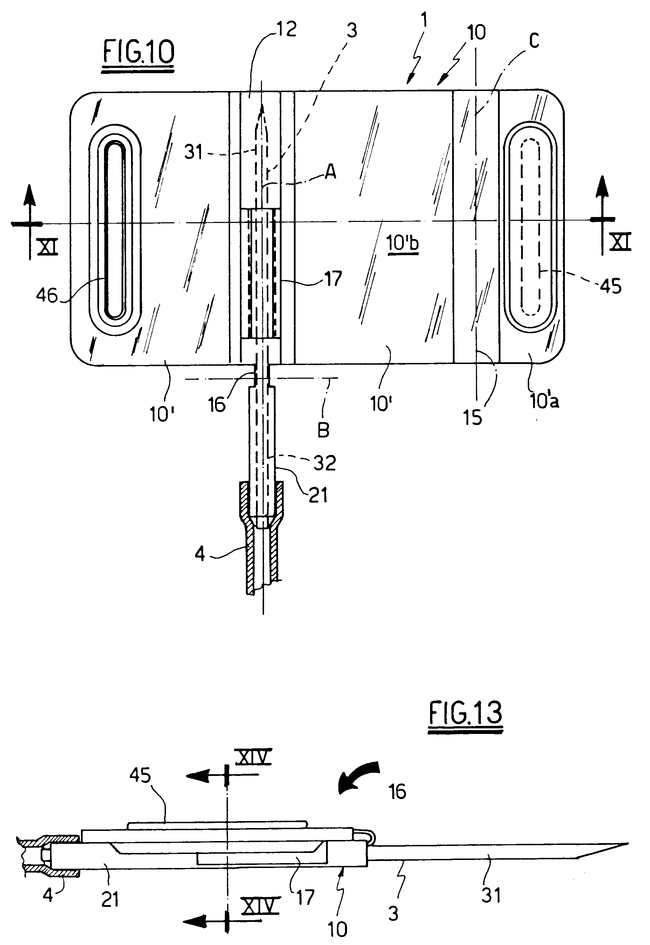

The device 1 can also assume a second operating configuration (Figures 7, 8, 9, 13 and 14) achieved by folding the element 10 relative to the needle 3 about the second axis B, in which the front portion 31 projects freely from the element 10. When in this second configuration the element 10 can be further folded on itself about the first axis (Figures 8, 9 and 14) so as to be able to be comfortably gripped between two fingers for its insertion into the body; alternatively it can be left open (Figures 7 and 13), to be rested on the surface of the body and fixed thereto by adhesive tape.

Preferably the element 10 is in the form of a thin plate sufficiently rigid to the extent that in normal use it cannot undergo deformation such as to enable the point of the needle 3 to project beyond the element 10 when in its first configuration. In practice this can be achieved by constructing the element 10 or indeed the entire device 1 of synthetic resin by a moulding process.

The rotation about the first axis A can be achieved by providing a suitable folding region 12 coaxial to the axis A, in which the thickness of the element 10 is reduced, the material with which the element 10 is formed being sufficiently flexible to allow bending along this line.

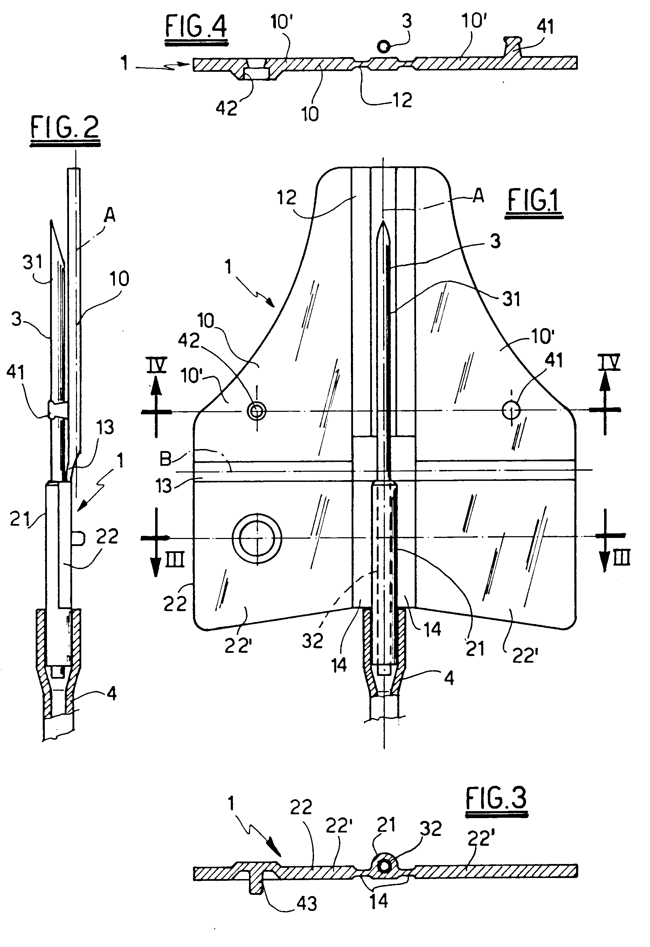

In the first embodiment, shown in Figures 1-9, said constraining means comprise a bush 21 which surrounds, and is rigidly joined to, the rear portion 32 of the needle 3, and a rear element 22 in the form of a thin plate which is joined to the bush 21 in a manner substantially coplanar therewith and is joined to the front element 10 in a manner substantially coplanar therewith by thin flexible portions 13. The rear element 22 is foldable on itself about an axis substantially coinciding with the first axis A; for this purpose it comprises a folding region 14 in the connection region between the element 22 and the bush 21. The flexible portions 13 enable the rear element 22, and hence the needle 3, to rotate relative to the front element 10 about the second axis B. The rear element 22 is divided into two lateral fins 22' by the folding region 14.

When the device 1 is folded into its second configuration, the front element 10 faces and is in contact with the rear element 22, whereas the front portion 31 of the needle 3 projects completely beyond the device 1 and can hence be inserted into the patient's body; the two elements 10 and 22 can be further folded together, by rotation about the axis A ie along the respective folding regions 12 and 14 which in this case are mutually superposed (Figure 9).

The folding regions 12 and 14 are each defined by two narrow regions of smaller thickness than the surrounding regions and located about a narrow central longitudinal portion parallel to the axis A and to which the bush 21 is joined to form a single piece therewith in the case of the element 22, whereas it faces the front portion 31 in the case of the element 10.

Means are provided for disengagably constraining to each other the two mutually facing fins 10' of the front element 10 when in the first operating configuration. Said means comprise one or more projections 41 positioned on the relative fins 10' and able to be press-inserted into corresponding cavities 42 provided in the opposing fin so as to provide a constraint of "snap-fastener" type. Figure 1 shows a projection 41 on the right fin 10' for insertion into a cavity 42 in the left fin 10' when the element 10 is folded along the axis A. Analogous constraining means can be provided for constraining the front element 10 to the rear element 22 when in the second operating configuration. Figure 3 shows a projection 43 for insertion into the same cavity 42, but from the opposite side with respect to the engagement with 41, when the two elements 10 and 22 face each other in mutual contact.

When in use, the device 1 is held in the first configuration when the front portion 31 has to be protected (to prevent pricking and to protect the integrity of the point), for example during the whole time in which the product is under distribution. To use the needle the projection 41 is firstly disengaged from the cavity 42 and the elements 10 and 22 are moved into a coplanar position (Figure 1); the element 10 is then folded about the axis B to bring it into the second operating configuration and allow the front portion 31 to project freely.

After further folding the elements 10 and 22 about the axis A (Figures 8 and 9), the device 1 can be comfortably gripped to introduce the needle into the body. After this, the elements 10 and 22 can be opened (Figure 7) to be rested on the surface of the body. Finally, when the needle 3 has been extracted from the body, the element 10 can be returned to the first configuration to enclose the needle point, after which the device 1 can be disposed of together with the needle, in the refuse. All the described operations can be comfortably and easily effected without the operator's fingers touching the needle, in particular its point, and hence with no danger of pricking. In addition when the needle 3 has been placed in the refuse it is protected by the device 1 and can be disposed off without danger of pricking other persons.

The entire device 1 can be advantageously constructed in one piece from synthetic resin by a moulding process.

Figures 10-14 show a second embodiment of the invention, which differs from the preceding by the following characteristics.

The rear element 22 is missing and the bush 21, fixed to the rear part 32 of the needle, is rigidly fixed to the front element 10 by a short, thin, flat flexible strip 16 which joins the front edge of the bush 21 to the rear edge of the front element 10, said strip 16 being substantially coaxial to the first axis A and being flexible to enable the needle 3 to be rotated with respect to the element 10 about the second axis B.

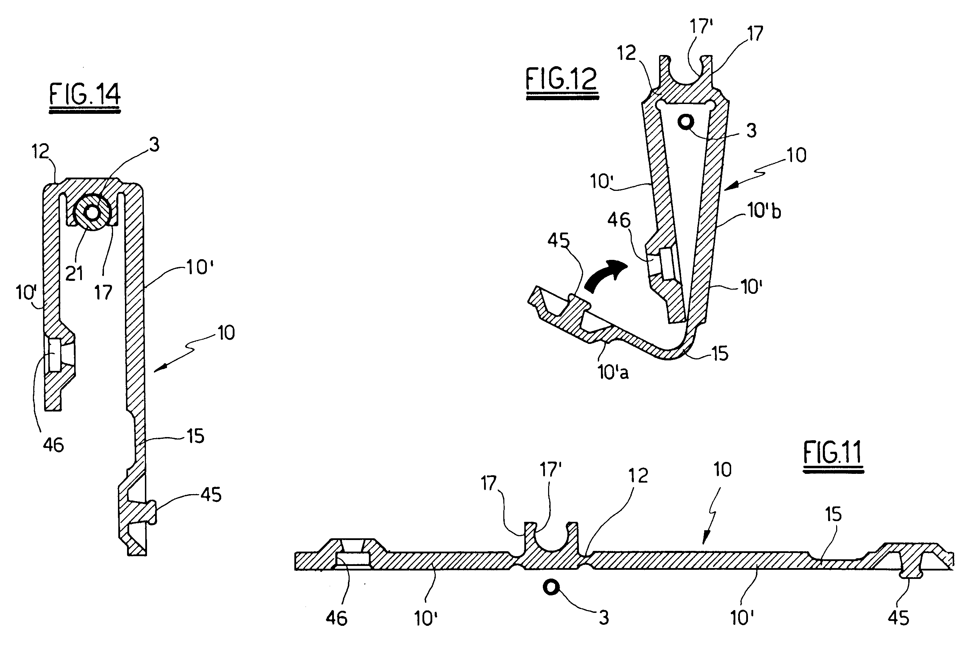

There is also provided a member 17 in one piece with the element 10 and having an axial channel 17' of circular arc cross-section extending parallel to and close to the first axis A. Said channel 17' projects outwards from that face of the element 10 opposite that which encloses the front portion 31 in the first configuration (Figure 12), the bush 21 being arranged to snap-penetrate into the channel 17' to be constrained thereto when the device 1 is in its second operating configuration (Figures 13 and 14).

The front element 10 (which in plan view can be of different form from that of Figure 1) also comprises a further folding region 15 defining a third folding axis C parallel to and spaced from the axis A; the region 15 divides a first fin 10' into an end portion 10'a and an intermediate portion 10'b. In the first operating configuration, the second fin 10' is folded about the axis A against the intermediate portion 10'b of the first fin 10', the end portion 10'a then being folded onto the second fin 10'. Constraining means are again provided, consisting for example of a projection 45 on the end portion 10'a and a corresponding cavity 46 in the second fin 10'to snap-receive the projection 45 and constrain the two fins 10' one to the other when in the first operating configuration. The projection 45 and cavity 46 are of elongate development in plan view.

To put the device 1 into its second operating configuration, after opening the element 10 (Figure 10) the element 10 is rotated with respect to the needle 3 about the strip 16 (axis B) to snap-insert the bush 21 into the channel 17' (Figure 13). After this, the element 10 can be folded about the axis A so that the fins 10' surround the rear portion 32 of the needle.

## Claims

1. A needle holding device for penetration into the body, such as a needle for intravenous infusion, dialysis and similar uses which comprises a front portion (31) and a rear portion (32); the device comprising a front element (10) having two fins (10'), positioned adjacent but not constrained to the front portion (31) of the needle (3) and means (21, 22, 13, 16) which constrain the rear portion (32) of the needle to the front element (10), characterised by that:
- said front element (10) is foldable on itself substantially about a first folding axis (A) parallel to the front portion (31) of the needle;
- said constraining means (21, 22, 13, 16) are such that the needle (3) can rotate with respect to the front element (10) about a second folding axis (B) substantially perpendicular to the needle (3);
- the device (1) being able to assume a first operating configuration in which the front element (10) is folded on itself by rotation along said first axis (A), with the front portion of the needle (3) enclosed between the two fins (10') of the element (10), these facing each other in mutual contact, the point of the needle (3) not projecting from the element (10);
- the device (1) being able to assume a second operating configuration, by rotating the front element (10) with respect to the needle (3) along said second axis (B), in which the front portion (31) of the needle freely projects from the front element (10); said front element (10) being formed from a thin plate sufficiently rigid to the extent that during normal needle use it cannot undergo deformation such as to enable the point of the needle (3) to project beyond the element (10) when in its first configuration.

2. A device as claimed in claim 1, characterised in that when in said second operating configuration, said front element (10) can be folded on itself about said first folding axis (A).

3. A device as claimed in claim 2, characterised in that said constraining means (21, 22, 13, 16) comprise a bush (21) which surrounds the rear portion (32) of the needle (3) and is rigidly fixed to this portion and to one or more flexile portions (13, 16) which connect it the front portion (10), said flexible portions (13, 16) enabling said rotation about said second folding axis (B).

4. A device as claimed in claim 1, characterised in that said front element (10) comprises a folding region (12) enabling rotation about said first axis (A), the front portion (31) of the needle (3) being positioned adjacent to said folding region (12).

5. A device as claimed in claim 3, characterised in that the constraining means comprises a rear element (22) in the form of a thin plate, joined to the bush (21) in a manner coplanar therewith and joined by said flexible portions (13) to the front element (10) in a manner substantially coplanar therewith, said rear element (22) being foldable on itself about an axis substantially coinciding with the first axis (A).

6. A device as claimed in claim 5, characterised in that when in said second configuration, the front element (10) faces and is in contact with the rear element (22), said elements (10, 22) being able to be further folded together about said first axis (A).

7. A device as claimed in claim 3, characterised by comprising a short, flat, flexible thin strip (16) which joins the front edge of the bush (21) to the rear edge of the front element (10), said strip (16) being positioned substantially coaxial to said first axis (A) and defining said flexible portion (13).

8. A device as claimed in claim 3, characterised by comprising a member (17) joined to the front element (10) and having a channel (17') extending parallel to said first axis (A), said channel (17') projecting outwards from that face of the front element (10) opposite the face which encloses the front portion (31) of the needle when in the first operating configuration, said bush (21) being arranged to snap-penetrate into the channel (17') when in the second operating configuration.

9. A device as claimed in claim 1, characterised by comprising means (41, 42, 45, 46) for disengagably constraining one to the other the two mutually facing fins (10') of the front element (10) when in its first operating configuration.

10. A device as claimed in claim 9, characterised in that said constraining means (41, 42, 45, 46) comprise one or more projections (41, 45) provided on relative fins (10') and able to be press-inserted into corresponding cavities (42, 46) provided in the opposing fin (10'), so as to form a constraint of "snap-fastener" type.

11. A device as claimed in claim 6, characterised by comprising constraining means (42, 43) for disengagably constraining one to the other the mutually facing front element (10) and rear element (22) when in said second operating configuration.

12. A device as claimed in claims 4 and 9, characterised in that said front element (10) comprises a further folding region (15) defining a third folding axis (C) parallel to but spaced from the first axis (A), said further folding region (15) defining in a first fin (10') an end portion (10'a) and an intermediate portion (10'b), said front element (10) being folded, when in the first operating configuration, such that the second fin (10') is in contact with the intermediate portion (10'b) of the first fin (10') and the end portion (10'a) is in contact with the second fin (10'), said constraining means (45, 46) being positioned between the end portion (10'a) and the second fin (10').

13. A device as claimed in claim 1, characterised by being constructed in one piece from synthetic resin by a moulding process.

## Patentansprüche

1. Nadelhalterungsvorrichtung zum Einstechen einer Nadel, welche einen vorderen Abschnitt (31) und einen hinteren Abschnitt (32) aufweist, in den Körper, insbesondere für intravenöse Infusionen, Dialyse und ähnliche Anwendungen; die Vorrichtung enthält ein Vorderteil (10) mit zwei Stegen (10'), die an dem vorderen Abschnitt (31) anliegen, jedoch nicht fest mit demselben verbunden sind und Einrichtungen (21,22,13,16), die den hinteren Abschnitt (32) der Nadel mit dem Vorderteil (10) fest verbinden,
**dadurch gekennzeichnet,** daß
- das Vorderteil (10) auf sich selbst im wesentlichen um eine erste Faltachse (A) parallel zum vorderen Abschnitt der Nadel faltbar ist;
- die Verbindungseinrichtungen (21,22,23,16) so gestaltet sind, daß die Nadel (3) in Bezug zum Vorderteil (10) um eine zweite Faltachse (B), die im wesentlichen senkrecht auf der Nadel (3) steht, rotieren kann;
- die Vorrichtung (1) eine erste Betriebsstellung einnehmen kann, in welcher das Vorderteil (10) durch Rotation um die erste Achse (A) auf sich selbst gefaltet wird, wobei der vordere Abschnitt der Nadel (3) zwischen die zwei Stege (10') des Vorderteils (10) eingeschlossen ist, wobei die Stege (10') sich gegenseitig berührend gegenüberstehen und die Spitze der Nadel nicht über das Teil (10) übersteht;
- die Vorrichtung eine zweite Betriebsstellung, in welcher die Nadel frei über das Vorderteil (10) herausragt, einnehmen kann, dadurch daß das Vorderteil (10) in Bezug zur Nadel (3) um die zweite Achse (B) rotiert wird; das Vorderteil (10) wird aus einer dünnen Platte gebildet, die in dem Maße ausreichend steif ist, daß sie während der normalen Nadelbenutzung keine Verformung erfahren kann, die es der Spitze der Nadel (3) erlaubt, in der der ersten Betriebsstellung über das Vorderteil (10) hinauszuragen.

2. Vorrichtung nach Anspruch 1,
**dadurch gekennzeichnet,** daß wenn sie sich in der zweiten Betriebsstellung befindet, das Vorderteil (10) um die erste Faltachse (A) auf sich selbst gefaltet werden kann.

3. Vorrichtung nach Anspruch 2,
**dadurch gekennzeichnet,** daß die Verbindungseinrichtungen (21,22,13,16) eine Buchse (21) beinhalten, welche den hinteren Abschnitt (32) der Nadel (3) umgibt und an diesem und einem oder mehreren flexiblen Abschnitten (13, 16) starr befestigt ist, die sie mit dem Vorderteil (10) verbinden und die Rotation um die zweite Faltachse (B) ermöglichen.

4. Vorrichtung nach Anspruch 1,
**dadurch gekennzeichnet,** daß das Vorderteil (10) einen Faltbereich (12) einschließt, der eine Rotation um die erste Achse (A) ermöglicht, wobei der vordere Abschnitt (31) der Nadel (3) direkt neben dem Faltbereich (12) angeordnet ist.

5. Vorrichtung nach Anspruch 3,
**dadurch gekennzeichnet,** daß die Verbindungseinrichtungen ein Hinterteil (22) in Form einer dünnen Platte einschließen, das mit der Buchse (21) in einer Ebene liegend verbunden ist und durch die flexiblen Abschnitte (13,16) im wesentlichen in einer Ebene mit dem Vorderteil (10) liegend mit diesem fest verbunden ist, wobei das Hinterteil (22) um eine Achse, die im wesentlichen mit der ersten Achse (A) zusammenfällt, auf sich selbst faltbar ist.

6. Vorrichtung nach Anspruch 5,
**dadurch gekennzeichnet,** daß wenn sie sich in der zweiten Stellung befindet, das Vorderteil (10) dem Hinterteil (22) gegenübersteht und es berührt, wobei die Teile (10,22) weiter um die erste Achse (A) zusammengefaltet werden können.

7. Vorrichtung nach Anspruch 3,
**gekennzeichnet durch** einen kurzen, flachen, biegsamen, dünnen Streifen (16), der das vordere Ende der Buchse (21) mit dem hinteren Ende des Vorderteiles (10) verbindet, wobei der Streifen im wesentlichen koaxial zu der ersten Achse (A) liegt und den flexiblen Abschnitt (13) bildet.

8. Vorrichtung nach Anspruch 3,
**gekennzeichnet durch** ein Bauelement (17), das mit dem Vorderteil (10) verbunden ist und eine Nut (17') parallel zu der ersten Achse (A) aufweist, wobei die Nut (17') von der Seite des Vorderteiles (10), die der Seite, welche in der ersten Betriebsstellung den vorderen Abschnitt (31) der Nadel einschließt gegenüberliegt, nach außen zeigt und die Buchse (21) so angeordnet ist, daß sie in der zweiten Betriebsstellung in der Nut (17') einschnappt.

9. Vorrichtung nach Anspruch 1,
**gekennzeichnet durch** Einrichtungen (41,42,45,46), mit denen die zwei sich gegenüberstehenden Stege (10') des Vorderteiles (10) in der ersten Betriebsstellung lösbar miteinander verbunden werden.

10. Vorrichtung nach Anspruch 9,
**dadurch gekennzeichnet,** daß die Verbindungseinrichtungen (41,42,45,46) eine oder mehrere an den Stegen (10') vorgesehene Auskragungen (41,45) enthalten, die in entsprechende Aussparungen (42,46), die auf dem gegenüberliegenden Steg (10') vorgesehen sind, eingedrückt werden und so eine Schnappverbindung bilden.

11. Vorrichtung nach Anspruch 6,
**gekennzeichnet durch** Verbindungseinrichtungen (42,43), mit denen das Vorderteil (10) und das Hinterteil (22), die einander gegenüberstehen, in der zweiten Betriebsstellung lösbar miteinander verbunden werden.

12. Vorrichtung nach den Ansprüchen 4 und 9,
**dadurch gekennzeichnet,** daß das Vorderteil (10) einen weiteren Faltbereich (15) einschließt, der eine dritte Faltachse (C) parallel versetzt zu der ersten Achse (A) definiert, wobei der Faltbereich (15) aus einem ersten Steg (10'), einem Endstück (10'a) und einem Zwischenstück (10'b) besteht und das Vorderteil (10) in der ersten Betriebsstellung so gefaltet wird, daß der zweite Steg (10') das Zwischenstück (10'b) des ersten Steges (10') berührt und das Endstück (10'a) den zweiten Steg (10') berührt, wobei sich die Verbindungseinrichtungen (45,46) zwischen dem Endstück (10'a) und dem zweiten Steg (10') befinden.

13. Vorrichtung nach Anspruch 1,
**dadurch gekennzeichnet,** daß sie durch einen Preßvorgang in einem Stück aus Kunstharz hergestellt wird.

## Revendications

1. Dispositif de maintien d'une aiguille destinée à pénétrer dans le corps, telle qu'une aiguille pour perfusions intraveineuses, dialyses et applications similaires qui comprend une partie frontale (31) et une partie arrière (32) ; le dispositif comprenant un élément frontal (10) comprenant deux ailettes (10') positionnées de façon adjacente mais n'étant pas retenue à la partie frontale (31) de l'aiguille (3) et des moyens (21, 22, 13, 16) qui retiennent la partie arrière (32) de l'aiguille à l'élément frontal 10, ledit dispositif étant caractérisé en ce que :
⇒ ledit élément frontal (10) peut être replié sur lui-même sensiblement autour d'un premier axe de pliage A parallèle à la partie frontale (31) de l'aiguille ;
⇒ lesdits moyens de retenue (21, 22, 13, 16) sont tels que l'aiguille (3) peut tourner par rapport à l'élément frontal (10) autour d'un second axe de pliage B sensiblement perpendiculaire à l'aiguille (3) ;
⇒ le dispositif (1) étant susceptible de prendre une première configuration opératoire dans laquelle l'élément frontal (10) est replié sur lui-même par rotation le long dudit premier axe A, avec la partie frontale de l'aiguille (3) contenue entre les deux aiguilles (10') de l'élément (10), celles ci se faisant face l'une l'autre en contact mutuel, la pointe de l'aiguille (3) ne faisant pas saillie de l'élément (10) ;
⇒ le dispositif (1) étant susceptible de prendre une seconde configuration opératoire, par rotation de l'élément frontal par rapport à l'aiguille (3) le long dudit second axe B, dans laquelle configuration la partie frontale (31) de l'aiguille fait saillie à partir de l'élément frontal (10) ; ledit élément frontal (10) étant formé à partir d'une plaque mince suffisamment rigide pour que, lors d'une utilisation normale de l'aiguille, il ne soit pas soumis à déformation de façon à permettre à la pointe de l'aiguille (3) de faire saillie au-delà de l'élément (10) lorsque le dispositif est dans sa première configuration.

2. Dispositif selon la revendication 1, caractérisé en ce que lorsqu'il est dans ladite seconde configuration opératoire, ledit élément frontal (10) peut être replié sur lui-même autour dudit premier axe de pliage (A).

3. Dispositif selon la revendication 2, caractérisé en ce que lesdits moyens de retenue (21, 22, 13, 16) comprennent un manchon (21) qui entoure la partie arrière (32) de l'aiguille (3) et est rigidement fixé à cette partie et à une ou plusieurs parties flexibles (13, 16) qui le relient à la partie frontale (10), lesdites parties flexibles (13, 16) permettant ladite rotation autour dudit second axe de pliage (B).

4. Dispositif selon la revendication 1, caractérisé en ce que ledit élément frontal (10) comprend une région de pliage (12) permettant une rotation autour dudit premier axe (A), la partie frontale (31) de l'aiguille (3) étant positionnée de façon adjacente à ladite région de pliage (12).

5. Dispositif selon la revendication 3, caractérisé en ce que les moyens de retenue comprennent un élément arrière (22) sous forme d'une plaque mince, reliée au manchon (21) de façon coplanaire avec lui et reliée par lesdites parties flexibles (13) à l'élément frontal (10) de façon sensiblement coplanaire avec lui, ledit élément arrière (22) pouvant être replié sur lui-même autour d'un axe coïncidant sensiblement avec le premier axe (A).

6. Dispositif selon la revendication 5, caractérisé en ce que lorsqu'il est dans ladite seconde configuration, l'élément frontal (10) fait face à l'élément arrière (22) et est en contact avec lui, lesdits éléments (10, 22) étant susceptibles d'être encore repliés ensemble autour dudit premier axe (A).

7. Dispositif selon la revendication 3, caractérisé en ce qu'il comprend une bande (16) courte, plate, flexible, mince qui relie le bord frontal du manchon (21) au bord arrière de l'élément frontal (10), ladite bande (16) étant positionnée sensiblement coaxialement audit premier axe (A) et définissant ladite partie flexible (13).

8. Dispositif selon la revendication 3, caractérisé en ce qu'il comprend un organe (17) réuni à l'élément frontal (10) et comportant un canal (17') s'étendant parallèlement audit premier axe (A), ledit canal (17') faisant saillie à l'extérieur à partir de cette face de l'élément frontal (10) qui est opposée à la face qui contient la partie frontale (31) de l'aiguille lorsque le dispositif est dans la première configuration opératoire, ledit manchon (21) étant disposé de façon à pénétrer en se clipsant dans le canal (17') lorsque le dispositif est dans la seconde configuration opératoire.

9. Dispositif selon la revendication 1, caractérisé en ce qu'il comprend des moyens (41, 42, 45, 46) pour retenir l'une à l'autre, tout en permettant leur désengagement, les deux ailettes (10') qui se font face mutuellement de l'élément frontal (10) lorsque le dispositif est placé dans sa première configuration opératoire.

10. Dispositif selon la revendication 9, caractérisé en ce que lesdits moyens de retenue (41, 42, 45, 46) comprennent une ou plusieurs saillies (41, 45) prévues sur les ailettes relatives (10') et qui sont susceptibles d'être introduits à pression dans des cavités correspondantes (42, 46) prévues dans l'ailette opposée (10'), de façon à constituer une retenue du type "bouton pression".

11. Dispositif selon la revendication 6, caractérisé en ce qu'il comprend des moyens de retenue (42, 43) pour retenir l'un à l'autre, tout en permettant leur désengagement, l'élément frontal (10) qui fait face mutuellement à l'élément arrière (22) lorsque le dispositif est placé dans ladite seconde configuration opératoire.

12. Dispositif selon les revendications 4 et 9, caractérisé en ce que ledit élément frontal (10) comprend une autre région de pliage (15) définissant un troisième axe de pliage (C) parallèle à, mais distant du premier axe (A), ladite autre région de pliage définissant dans une première ailette (10') une partie d'extrémité (10'a) et une partie intermédiaire (10'b), ledit élément frontal (10) étant replié, lorsque le dispositif est dans la première configuration opératoire, de telle façon que la seconde ailette (10') soit en contact avec la partie intermédiaire (10'b) de la première ailette (10') et que la partie d'extrémité (10'a) soit en contact avec la seconde ailette (10'), lesdits moyens de retenue (45, 46) étant positionnés entre la partie d'extrémité (10'a) et la seconde ailette (10').

13. Dispositif selon la revendication 1, caractérisé en ce qu'il est construit en une seule pièce à partir d'une résine synthétique par un procédé de moulage.
